# EUROPEAN PATENT SPECIFICATION

(11) **EP 3 602 165 B1**
(45) Date of publication and mention of the grant of the patent: **04.11.2026**
(21) Application number: 18770930.8
(22) Date of filing: 19.03.2018
(51) Int. Cl.: G02B 21/00, G02B 21/36, G02B 27/64, A61B 3/13, A61B 3/135, A61B 3/10

(54) **OPTICAL ADAPTER FOR MICROSCOPE AND METHOD FOR ADJUSTING DIRECTION OF OPTICAL IMAGE**
OPTISCHER ADAPTER FÜR MIKROSKOP UND VERFAHREN ZUR EINSTELLUNG DER RICHTUNG EINES OPTISCHEN BILDES
ADAPTATEUR OPTIQUE POUR MICROSCOPE ET PROCÉDÉ DE RÉGLAGE DE DIRECTION D'IMAGE OPTIQUE

(30) Priority: 21.03.2017 CN 201710168851; 13.04.2017 CN 201710238871
(43) Date of publication of application: 05.02.2020
(73) Proprietor: Zumax Medical Co., Ltd., Jiangsu 215129 (CN)
(72) Inventor: LI, Jianyue, Suzhou Jiangsu 215129 (CN); YANG, Xiaoguang, Suzhou Jiangsu 215129 (CN); DU, Lei, Suzhou Jiangsu 215129 (CN); HUANG, Bin, Suzhou Jiangsu 215129 (CN); ZHANG, Quanwu, Suzhou Jiangsu 215129 (CN)
(74) Representative: Zoli, Filippo
(86) International application number: PCT/CN2018/079406
(87) International publication number: WO 2018/171537

(56) References cited:
- EP-A2- 1 804 104
- CN-A- 105 264 435
- CN-A- 106 940 472
- CN-A- 107 807 442
- CN-U- 204 188 876
- CN-U- 205 507 214
- CN-U- 206 618 896
- CN-Y- 201 203 701
- US-A- 6 072 625
- US-B2- 9 438 773

## Description

This application claims the priority benefit of CN Application No. 201710168851.0 filed on March 21, 2017; and CN Application No. 201710238871.0 filed on April 13, 2017,

### FIELD OF THE INVENTION

The present invention generally relates to medical devices and equipment, but not by way of limitation, to optical adapters for microscopes.

### BACKGROUND OF THE DISCLOSURE

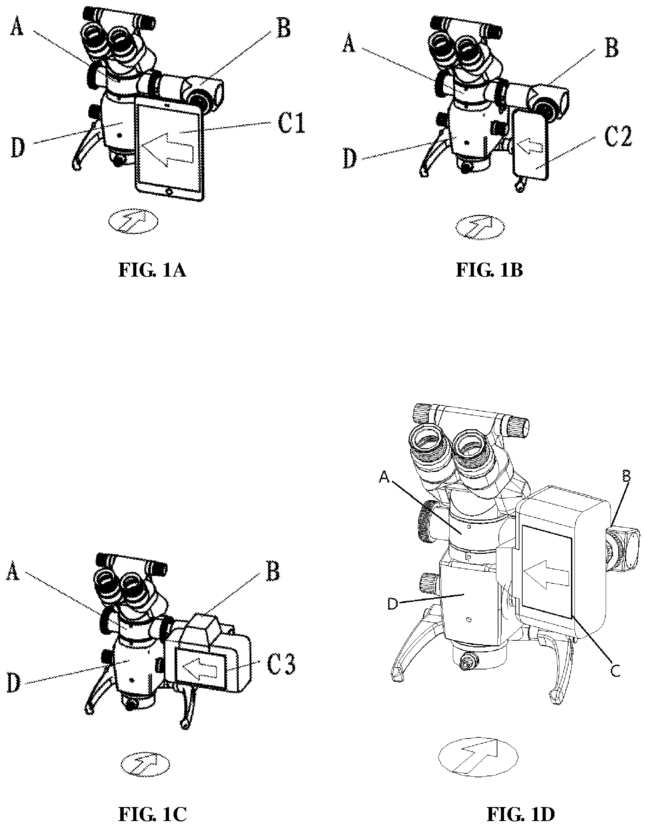

As shown in FIGS. 1A, 1B, and 1C, a conventional optical adapter B is used for connecting a beam splitter A from a microscope D to a digital camera equipment C1/C2/C3. The optical adapter B is configured to be rotatable around an axis of rotation so that it may be positioned in different rotational positions, usually 90 degrees between each position, thereby adjusting the direction of digital camera equipment C1, C2, and C3 in upward, forward, downward, or backward positions. This is performed by selecting the direction of the mount when connected to the beam splitter A, and by changing the angle required for reassembling.

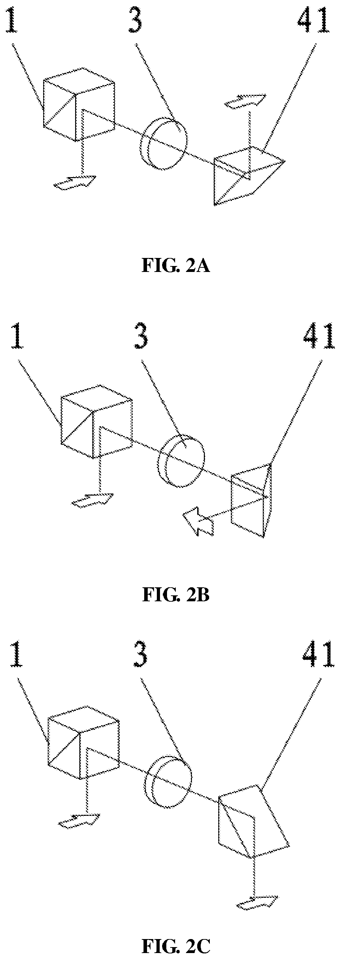

An example of the conventional optical adapter B and its optical path is shown schematically in FIGS. 2A, 2B, and 2C. As it can be seen from these figures, the lens 3 and the right angle prism 41 (plane mirror is also acceptable) are sequentially arranged in the optical adapter B. When the optical adapter B is rotated to a different position, the reflective surface of the right angle prism 41 rotates about the optical axis. As such, the normal of the splitting surface of the beam splitter A and the normal of the reflective surface of the right angle prism 41 no longer remain parallel, thereby, the direction of the optical image will be rotated. For example, if the optical adapter B is in its initial position, i.e., the right angle lens does not rotate, and thus, the resulting optical image is as shown in FIG. 2A. When the optical adapter B rotates to the next gear, the reflective surface of the right angle prism 41 will rotate by 90 degrees, and the resulting optical image is as shown in FIG. 2B. Alternatively, the reflective surface of the right angle prism 41 rotates by 180 degrees when the optical adapter B further rotates to the next gear, and the resulting optical image is as shown in FIG. 2C.

Since the photosensitive unit of digital camera equipment is rectangular, and the output signals from the digital camera are sequentially set, there is going to be different image directions including: up, down, left and right. Consequently, images captured by the digital camera equipment need to be uploaded to a computer or a network for viewing, saving, editing and the like. Therefore, images with a wrong direction are not convenient for use and they need to be adjusted by a video software (with only 90 degrees multiple rotations) , as shown in FIGS. 1A, 1B, and 1C. Therefore, there is a constant need to re-adjust the installation direction of digital camera equipment so as to rotate the photosensitive unit in an appropriate orientation, to ensure that the images captured by the photosensitive unit are in accordance with the direction of images observed in the microscope. Even so, there may still exist some inconsistency between the image displayed on the display of the digital camera equipment and the normal viewing direction of the operator, resulting in great inconvenience of use, e.g., FIG. 1 D.

Moreover, the digital camera C used in the mobile phones or the tablet devices captures images by focusing on the beam splitter A of the microscope D or uses a dedicated adapter in the camera (FIGS. 1A-1D) . This may cause the captured images to be cut or not being able to fill the entire field of view of the digital camera due to existence of a mismatch between the angle of view and the pupil. Consequently, a user may have to manually zoom-in on the mobile phone or the tablet device to enlarge the image, which results in loss of information and image quality and further complex operations.
Devices of the known type are disclosed by EP 1 804 104 A2, CN 201 203 701 Y, CN 204 188 876 U and US 6 072 625 A. More in particular, CN 201 203 701 Y and EP 1 804 104 A2 disclose an optical adapter of the known type for connecting between a beam splitter of a microscope and a digital camera equipment, CN 204 188 876 U discloses a rotating lens unit in a surgical microscope and US 6 072 625 A discloses a confocal scanning type optical microscope apparatus including an image rotation device.

### SUMMARY OF THE INVENTION

The present invention as defined in the appended independent claims 1, 13 and 15 is proposed in view of the above aforementioned problems.

In one embodiment, the optical adapter may include a body housing, where one end of the body housing is connected to the beam splitter of the microscope, and the other end of the body housing is connected to the digital camera equipment.

In another embodiment, the lens group may include at least a first lens unit for converging an outgoing light of the beam splitter into a real image, and a second lens unit for projecting the real image on the photosensitive unit of the digital camera equipment, the second lens unit being disposed at one end of the lens group. In this embodiment, an exit pupil of said lens group is located behind the second lens unit, and a distance between the exit pupil and an apex located on the rear optical surface of a final lens in said second lens unit is not more than 30mm. Preferably, the first lens unit and the second lens unit are convex lens unit, and a focal length of the first lens unit is greater than a focal length of the second lens unit while a ratio of focal length between the first lens unit and the second lens unit is greater than 3.

In a preferred embodiment, the optical image rotating unit is disposed within the body housing such that the optical image rotating lens group and the lens group are both disposed in the body housing. In this way, all the optical elements are located within the body housing.

In this preferred embodiment, a rotating part is disposed on the body housing for rotating the optical image rotating lens group, the optical image rotating lens group is connected to the rotating part, the rotation of the optical image rotating lens group can be facilitated by the provision of the rotating part on the body housing, the rotating part may be a rotating joint on the body housing or the like.

In an alternative embodiment, a scale for indicating a rotational angle of the rotating part may be provided on the body housing and/or on the rotating part. In this way, the optical image rotating lens group can operate accurately using the scale, so that the rotation of optical image rotating lens group in the right direction can be easily facilitated.

In yet another embodiment, the optical rotating unit is connected to an outside of the body housing. In this embodiment, the optical rotating unit is connected to an outside of the body housing as a relatively independent component, and can be connected with the body housing fixedly or detachably. In this embodiment, except for the optical image rotating lens group, the lens group and/or other optical elements are still disposed within the body housing.

In yet another embodiment, the optical image rotating unit may be connected to one end of the body housing. For example, when the optical adapter is connected to the beam splitter the optical image rotating unit is adapted to be disposed between the beam splitter and the one end of the body housing. Or alternatively, the optical image rotating unit may be connected to the other end of the body housing. In this case, when the optical adapter is connected to the digital camera equipment, the optical image rotating unit is adapted to be disposed between the other end of the body housing and the digital camera equipment. In this way, the optical image rotating unit operates in two connection modes by being able to connect to both ends of the body housing.

The optical image rotating lens group include at least one of the Dove prism, Dove roof prism, Pechan prism, Pechan roof prism and a Right angle prism.

In yet another embodiment, the optical adapter further includes a group of mirrors located on the optical path, where the group of mirrors can be disposed within the body housing. This group of mirrors may include at least one reflecting element having at least one of the reflector, pentaprism, right angle prism and right angle roof prism.

In yet another embodiment, the group of mirrors may include at least two reflecting elements that reflect the optical images at least two times. In this way, the direction of optical image on the photosensitive unit can be adjusted in two dimensions. Similarly, the number of reflecting elements may be increased sequentially to achieve multidimensional adjustment of the optical image.

In a preferred embodiment, the optical adapter may further include the digital camera equipment. In this embodiment, the digital camera equipment becomes an integral part of the optical adapter.

In accordance with an aspect of the present invention, there is provided a process for adjusting a direction of an optical image using a microscope optical adapter in a microscope, the method includes the steps of: connecting the microscope optical adapter with a beam splitter of the microscope on its light incident end; connecting a digital camera equipment to the light emitting end of said microscope optical adapter; forming an optical image on a photosensitive unit of the digital camera equipment. The microscope optical adapter includes an optical image rotating unit for adjusting a direction of an optical image on a photosensitive unit of the digital camera equipment. The optical image rotating unit includes an optical image rotating lens group which is configured to be rotatable around optical axis independently with respect to the beam splitter. When a light emitting direction of the microscopic optical adapter is changed by rotation of the optical image rotating unit, the optical image formed by the light emitted from different emission directions on the photosensitive unit of the digital camera equipment can be aligned with the image observed by the microscope without an adjustment of computer software. The optical image rotation lens group comprises a plurality of Right angle prisms, and an orientation of the optical image is adjusted by rotating a same relative rotation angle between adjacent Right angle prisms.

By way of comparison with prior art, embodiments of the present invention provides the following advantages and effects as a result of the application of the above technical solutions.

Embodiments of the present invention provide for a system and method for real-time adjustment of the direction of an optical image on the photosensitive unit of a digital camera equipment, where regardless of the position of the digital camera a satisfactory direction of the optical image can be obtained with a simple user friendly and convenient structure.

### BRIEF DESCRIPTION OF THE DRAWINGS

The present disclosure is described in conjunction with the appended figures:
FIGS. 1A, 1B, 1C, and 1D illustrate an exemplary schematic diagram of a prior art optical adapter and a microscope.
FIGS. 2A, 2B, and 2C illustrate an exemplary schematic diagram of an optical path of optical elements existing in the prior art optical adapters.
FIG. 3-5, 7-9 illustrate schematic diagrams of exemplifying optical paths of optical adapters.
FIG. 6, 10 and 11 illustrate schematic diagrams of optical paths of optical adapters according to embodiments of the present invention.
FIG. 12 illustrates a cross-sectional view of an exemplary optical adapter for a microscope.
FIG. 13 illustrates a schematic diagram of exemplary optical path of an optical adapter.
FIGS. 14A, 14B, and 14C illustrate an exemplary schematic diagram of an optical adapter according to the embodiments of the present invention and a microscope.

In the appended figures, similar components and/or features may have the same reference label. Further, various components of the same type may be distinguished by following the reference label by a dash and a second label that distinguishes among the similar components. If only the first reference label is used in the specification, the description is applicable to any one the similar components having the same first reference label irrespective of the second reference label. For ease of understanding, the main components are listed below with their corresponding reference labels: 1: beam splitter prism; 20: Dove prism; 21: Dove roof prism; 22: Pechan prism; 23: Pechan roof prism; 3, 30, 31: lens group; 310: rear optical surface; 40: pentaprism; 41: right angle prism; 42: right angle roof prism; 5: body housing; 50: first body housing; 51: second body housing; 52: rotating part; A: beam splitter; B: optical adapter; C: digital camera; C1: tablet computer; C2: mobile phone; C3: camera; D: microscope; E: real image; F: apex; G: exit pupil.

### DETAILED DESCRIPTION OF THE INVENTION

The present invention will be further described below with reference to the accompanying drawings and embodiments. As will be described further below, the optical image rotating lens group, as set forth in the embodiments I-IV, may include Dove prism or Dove roof prism which rotate around the optical axis to adjust the orientation of optical images captured by the digital camera. In order to avoid image mirroring caused by odd number of total reflections, a combination of prisms may be used in the following manners.

### Example

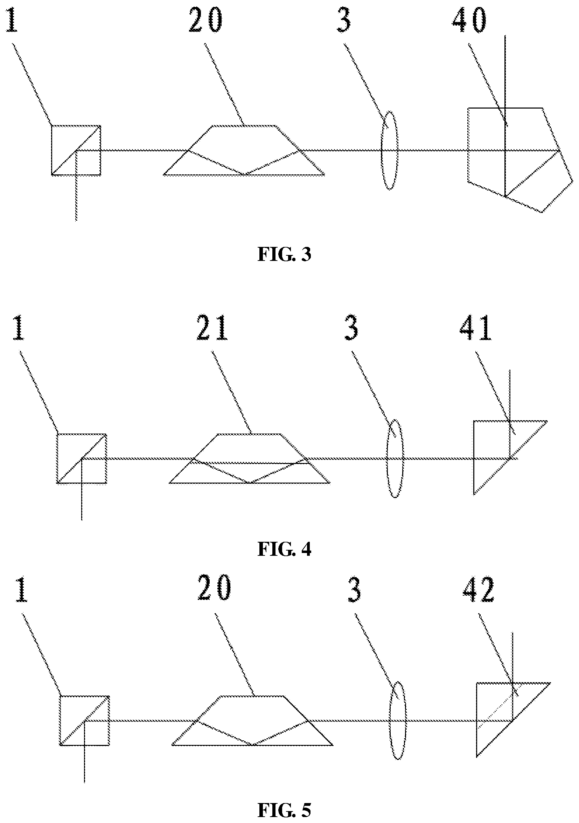

Referring first to FIG. 3, an optical path of an optical adapter for a microscope is shown. The optical adapter may include a Dove prism 20, a lens group 3 and a pentaprism 40 which are sequentially arranged on one splitting optical path of the beam splitter prism 1. As it can be seen form Fig. 3, the incident light is reflecting once inside the Dove prism 20 and twice inside the pentaprism 40 to ensure a total number of four times reflections. In this example, the orientation of optical images formed on the photosensitive unit of the digital camera equipment C1, C2, and C3 can be adjusted by only rotating the Dove prism 20. This combination of prisms allows for avoiding a higher cost and more complicated process that are used in manufacturing of roof prisms.

### Example II

Referring next to FIG. 4, an optical path of an optical adapter for a microscope is shown. In this example, the optical adapter includes a Dove roof prism 21, the lens group 3 and a right angle prism 41 which are sequentially arranged on the splitting optical path of the beam splitter prism 1. In this example, the incident light is reflecting twice inside the Dove roof prism 21 and once inside the right angle prism 41 to ensure a total number of even reflections (four times). Similar to the first example, the rotation of Dove roof prism 21 alone can adjust the orientation of the optical images formed on the photosensitive unit of the digital camera equipment C1, C2, and C3.

### Example III

With reference to FIG. 5, an optical path of an optical adapter for a microscope is shown. In this example, the optical adapter includes a Dove prism 20, the lens group 3 and a right angle roof prism 42 which are sequentially arranged on the splitting optical path of the beam splitter prism 1. In this example, the incident light is reflecting once inside the Dove prism 20 and twice inside the right angle roof prism 42 to ensure a total number of even reflections (four times). Similar to the first example, the rotation of Dove roof prism 20 alone can adjust the orientation of the optical images formed on the photosensitive unit of the digital camera equipment C1, C2, and C3.

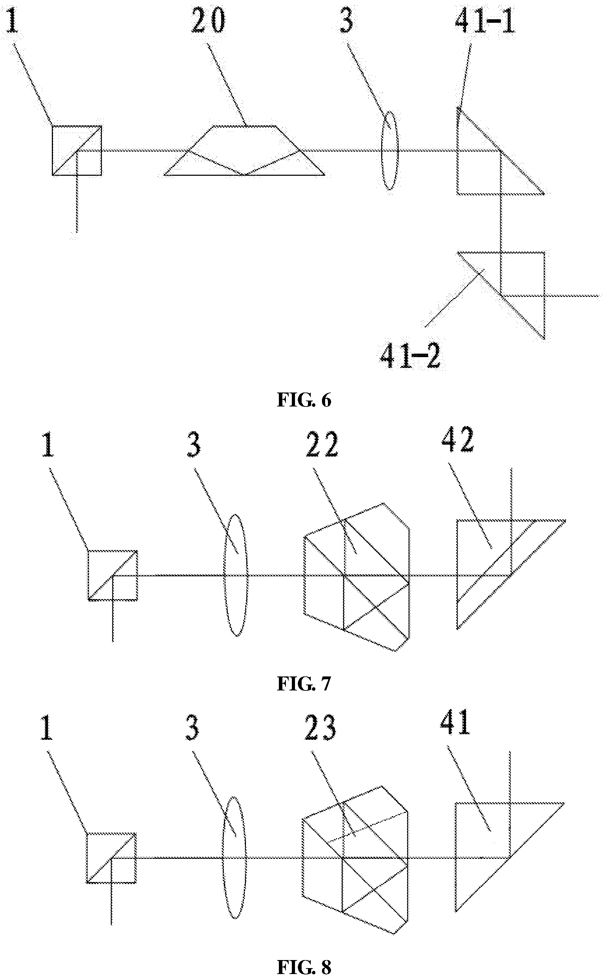

FIG. 6 illustrates an optical path of an optical adapter for a microscope according to an embodiment of the present invention.

The pentaprism 40 is replaced by two right angle prisms 41 (reference labels 41-1 &41-2 in FIG. 6) . In this embodiment, the incident light is reflecting once inside the Dove prism 20 and once inside each of the right angle prisms 41 to ensure a total number of even reflections (four times) . This configuration also allows for avoiding a higher cost and more complicated process that are used in manufacturing of roof prisms. Similar to the previous examples, the optical elements are sequentially arranged on the splitting optical path of the beam splitter prism 1. Using two right angle prisms 41 (reference labels 41-1 &41-2 in FIG. 6) allows for two-dimensional adjustment of the optical image. Similarly, a multidimensional adjustment of the optical image can be achieved by adding more reflections. Here again the rotation of Dove prism 20 alone can adjust the orientation of the optical images formed on the photosensitive unit of the digital camera equipment C1, C2, and C3.

The optical image rotating lens group, as set forth in the embodiments V-VIII, may include Pechan prism or Pechan roof prism which rotate around the optical axis to adjust the orientation of optical images captured by the digital camera. The optical path of optical elements in the embodiments V-VIII is different from the previous embodiments I-IV, in that the lens group 3 is located as the first optical element of the optical path. In order to avoid image mirroring caused by odd number of total reflections, a combination of prisms may be used in the following manners.

### Example IV

Referring next to FIG. 7, an optical path of an optical adapter for a microscope is shown. The optical adapter may include the lens group 3, a Pechan prism 22 and a right angle roof prism 42 which are sequentially arranged on the splitting optical path of the beam splitter prism 1. As it can be seen form Fig. 7, the incident light is reflecting five times inside the Pechan prism 22 and twice inside the right angle roof prism 42 to ensure a total number of eight times reflections. Here again, the orientation of optical images formed on the photosensitive unit of the digital camera equipment C1, C2, and C3 can be adjusted by only rotating the Pechan prism 22.

### Example V

FIG. 8 illustrates an optical path of an optical adapter for a microscope. In this example, the optical adapter may include the lens group 3, a Pechan roof prism 23 and a right angle prism 41. The optical elements are sequentially arranged on the splitting optical path of the beam splitter prism 1 with the lens group 3 being disposed as the first optical element of the optical path. In this example, the incident light is reflecting six times inside the Pechan roof prism 23 and once inside the right angle prism 41 to ensure a total number of even reflections (eight times) . Here again, the rotation of Pechan roof prism 23 alone can adjust the orientation of the optical images formed on the photographic unit of the digital camera equipment C1, C2, and C3.

### Example VI

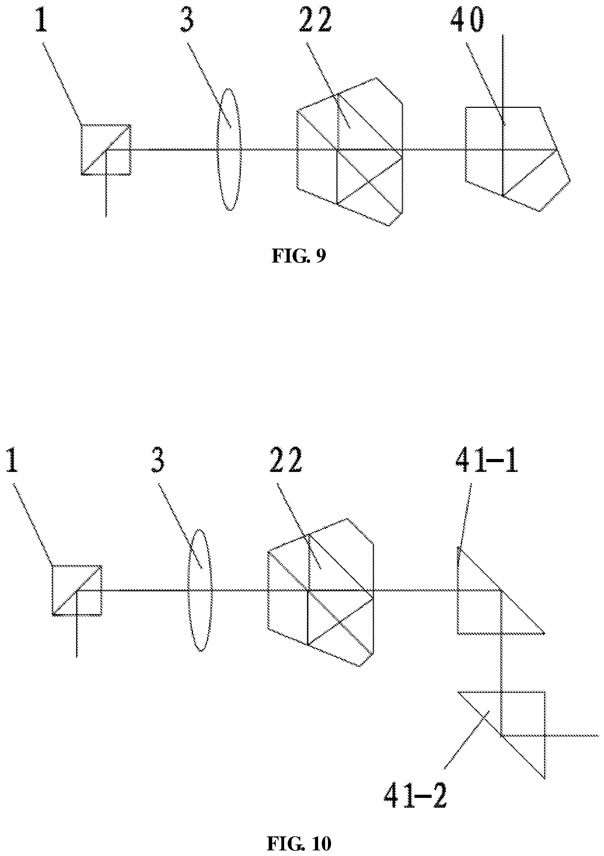

With reference to FIG. 9, an optical path of an optical adapter for a microscope is shown. In this example, the optical adapter may include the lens group 3, a Pechan prism 22 and a pentaprism prism 40 which are sequentially arranged on the splitting optical path of the beam splitter prism 1 with the lens group 3 being disposed as the first optical element of the optical path. In this embodiment, the incident light is reflecting five times inside the Pechan prism 22 and twice inside the pentaprism 40 to ensure a total number of even reflections (eight times) . This combination of prisms allows for avoiding a higher cost and more complicated process that are used in manufacturing of roof prisms. Similar to the previous examples, the rotation of Pechan prism 22 alone can adjust the orientation of the optical images formed on the photosensitive unit of the digital camera equipment C1, C2, and C3.

FIG. 10 illustrates an optical path of an optical adapter for a microscope according to an embodiment of the present invention. The right angle roof prisms 42 is replaced by two right angle prisms 41 (reference labels 41-1 &41-2 in FIG. 10) . In this embodiment, the incident light is reflecting five times inside the Pechan prism 22 and once inside each of the right angle prisms 41 to ensure a total number of even reflections (eight times). This configuration also allows for avoiding a higher cost and more complicated process that are used in manufacturing of roof prisms. Here again, the optical elements of the eighth embodiment are sequentially arranged on the splitting optical path of the beam splitter prism 1 with the lens group 3 being disposed as the first optical element of the optical path. Using two right angle prisms 41 (reference labels 41-1 &41-2 in FIG. 10) allows for two-dimensional adjustment of the optical image. Similarly, a multidimensional adjustment of the optical image can be achieved by adding more reflections. Here again the rotation of Pechan prism 22 alone can adjust the orientation of the optical images formed on the photosensitive unit of the digital camera equipment C1, C2, and C3.

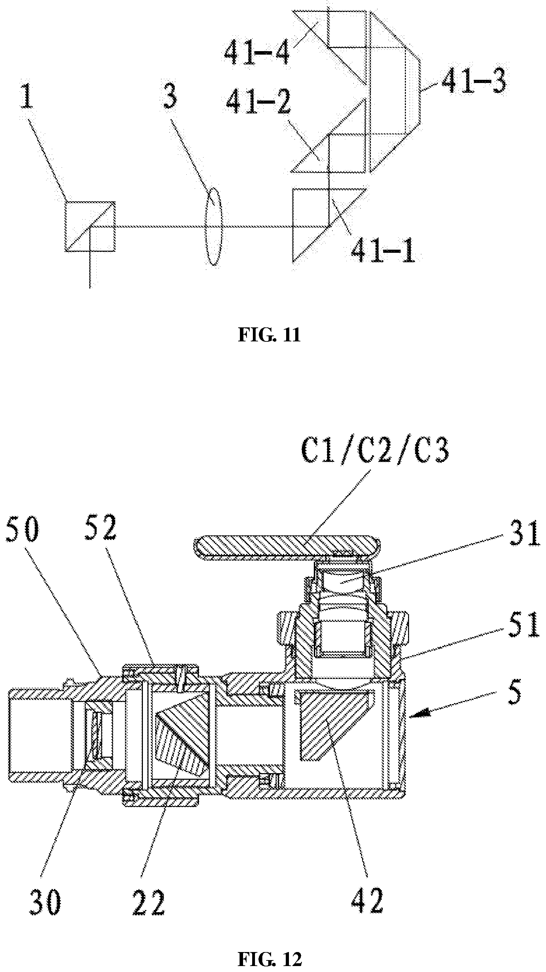

Referring next to FIG. 11, an exemplary optical path of an optical adapter for a microscope according to an embodiment of the present invention is shown. In this embodiment, the optical adapter may include the lens group 3 and a group of mirrors which are sequentially arranged on the splitting optical path of the beam splitter prism 1 with the lens group 3 being disposed as the first optical element of the optical path. As can be seen from FIG. 11, the group of mirrors includes four right angle prisms 41 (reference labels 41-1, 41-2, 41-3 &41-4) where the optical image rotating lens group contains three right angle prisms 41 (reference labels 41-2, 41-3, &41-4 in FIG. 11) at the end of the optical path. In this embodiment, the right angle prism 41-4 can follow the rotation of digital camera equipment while the relative rotational angle between the right angle prism 41-4 and the right angle prism 41-3 remains always the same as the relative rotational angle between the right angle prism 41-2 and the right angle prism 41-3. This is achieved by a mechanical linkage which allows for continuously adjusting the orientation of digital camera equipment C1, C2, and C3 so as to automatically maintain the direction of optical images constant.

FIG. 12 illustrates a cross-sectional view of an example of an optical adapter for a microscope. As shown in this figure, the optical adapter may include an L-shaped body housing 5, where one end of the body housing 5 is configured to connect to the beam splitter A, while the other end of the body housing 5 is configured to connect to the digital camera equipment C1, C2, and C3. In this embodiment, the lens group may include a first lens unit 30 and a second lens unit 31. Inside the body housing 5, a first lens unit 30, a Pechan prism 22, a right angle roof prism 42, and a second lens unit 31 are sequentially arranged on the splitting optical path of the beam splitter prism from the beam splitter. Here again the rotation of the Pechan prism 22 alone can adjust the orientation of the optical images formed on the photosensitive unit of the digital camera equipment C1, C2, and C3.

In this example, the L-shaped body housing 5 further includes a first body housing 50 for connecting to the beam splitter A of the microscope and a second body housing 51 for connecting to the digital camera equipment C1, C2, and C3. The first body housing 50 is set vertically relative to the second body housing 51, and the second body housing 51 can perform a 360-degree stepless rotation relative to the first body housing 50, allowing a stepless adjustment of the positioning of the digital camera equipment C1, C2, and C3. The first body housing 50 and/or the second body housing 51 is provided with a scale for indicating the rotation angle of the second body housing 51.

In order to facilitate the rotation of the Pechan prism 22, the body housing 5 is further provided with a rotating part 52 which is disposed on a top surface of the first body housing 50. As shown in Fig. 12, the Pechan prism 22 is connected with the rotating part 52 such that the rotation of the Pechan prism 22 about the optical axis can be achieved by operating the rotating part 52. In addition, the body housing 5 and/or the rotating part 52 may be provided with another scale for indicating a rotation angle of the rotating part 52. This scale is configured to match the scale of the second body housing 51 such that the rotating part 52 can operate quickly and accurately so as to facilitate the rotation of the Pechan prism 22.

In an embodiment of the invention, a lens group, an optical image rotating lens group and a group of mirrors may be additionally disposed within the body housing 5 of the optical adapter B. In this embodiment, according to different combinations of the lens group, the optical image rotating lens group and the group of mirrors, the optical image rotating lens group may be disposed between the beam splitter A and the body housing 5 of the optical adapter B, or between the body housing 5 of the optical adapter B and the digital camera equipment C1, C2, and C3.

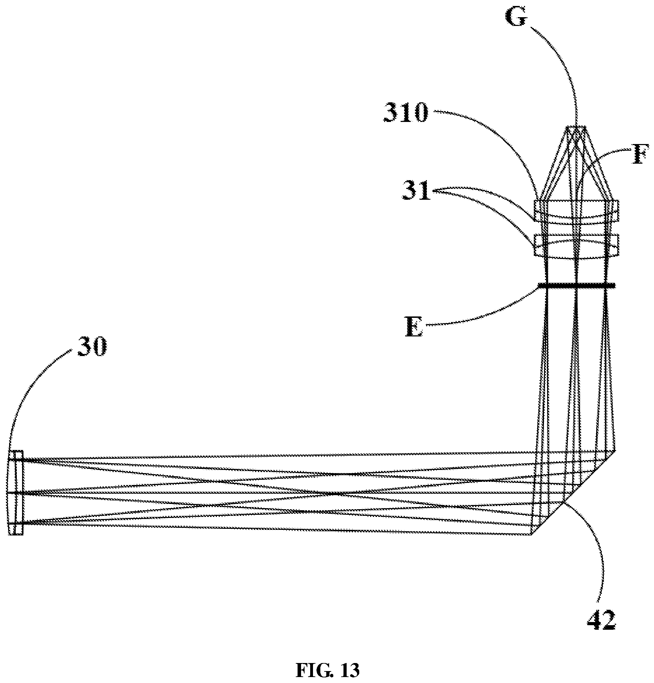

With reference to FIG. 13, an optical path of the optical adapter according to an example is shown. As described further above, the lens group of the optical adapter of the present embodiment may include a first lens unit 30 and a second lens unit 31. The first lens unit 30 which is located as the first optical element of the optical path and is used for converging an outgoing light of the beam splitter A into a real image E. The second lens unit 31 which are located as the last element of the optical path at the other end of the optical adapter and is used for projecting the real image E on the photosensitive unit of the digital camera equipment C1/C2/C3. As shown in FIG. 13, an exit pupil G of the lens group is located behind the second lens unit 31, and a distance between the exit pupil G and an apex F located on a rear optical surface 310 of the last or final lens in the second lens unit 31 is not more than 30mm.

More specifically, since the distance between an entrance pupil of a micro camera (the digital camera equipment C1/C2/C3) and the rear optical surface 310 from the last lens of the second lens unit 31 is generally less than 10 mm, the distance of the exit pupil G to the rear optical surface 310 of the present embodiment does not exceed more than 30 mm to ensure adjustability, and therefore, matching with the entrance pupil of the micro camera. The imaging range of the micro camera on a mobile phone is generally 45 degrees X 60 degrees (rectangular light sensing unit) , where after being amplified 8-10 times by the optical adapter described in the present embodiment, electronic zoom-in is no longer required, and the optical performance of the micro camera can be fully utilized.

The first lens unit 30 and the second lens unit 31 are convex lens units where a focal length of the first lens unit 30 is greater than a focal length of the second lens unit 31. More preferably, a ratio of focal length between the first lens unit 30 and the second lens unit 31 is greater than 3. Accordingly, a corresponding focusing mechanism may also be required to achieve the above-mentioned focal length adjustment between the first lens group 30 and the second lens group 31. In addition, each of the first lens unit 30 and the second lens unit 31 may further include one or more of an iris, a color filter and a polarizer. In this way, the size of the light aperture may be adjusted freely by setting the iris, the shape of the light may be kept approximately circular, and the stray light can be eliminated by the color filter and the polarizer.

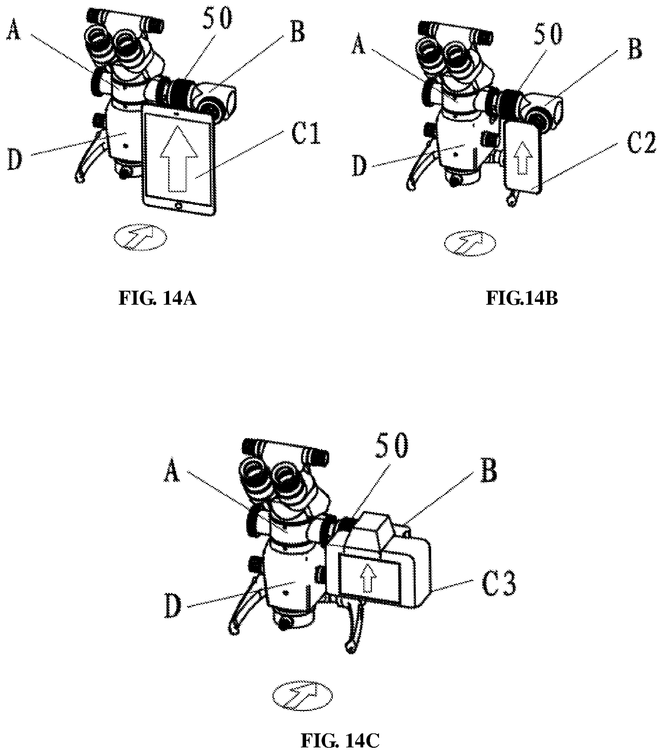

By configuring the optical image rotating lens group as described in the above-mentioned examples, the direction of the optical image can be adjusted by independently rotating the optical image rotating lens group to ensure the most appropriate image observation in the microscope, regardless of positioning of the digital camera equipment C1, C2, C3, e.g., facing upward, downward, forward or backward. Please refer to the comparison between FIGS. 1A, 1B, 1C and FIGS. 14A, 14B, 14C.

## Claims

1. An optical adapter (B) for connecting between a beam splitter (A) of a surgical microscope or an ophthalmic slit lamp microscope and a digital camera equipment (C), the optical adapter (B) comprising:
a lens group (3) located on an optical path; and
an optical image rotating unit configured to adjust a direction of an optical image on a photosensitive unit of the digital camera equipment (C),
**characterized in that** said optical image rotating unit comprises a plurality of right angle prisms (41) being four right angle prisms (41-2, 41-3, 41-4) located on the optical path, wherein said plurality of right angle prisms are configured to be rotatable around optical axis independently with respect to the beam splitter;
wherein, an orientation of the optical image is adjusted by rotating the plurality of right angle prisms while maintaining a same relative rotation angle between adjacent right angle prisms..

2. The optical adapter of claim 1, **characterized in that** the optical adapter (B) further comprising a body housing (5) encompassing the lens group (3), wherein one end of the body housing (5) connecting to the beam splitter (A) while another end of the body housing (5) being connected to the digital camera equipment (C).

3. The optical adapter of claim 1, **characterized in that** the lens group (3) comprises at least a first lens unit for converging an outgoing light of the beam splitter (A) into a real image (E), and a second lens unit for projecting said real image (E) on the photosensitive unit of the digital camera equipment (C), said second lens unit being disposed at an opposite end of the optical path compared to said first lens unit, wherein an exit pupil (G) of said lens group (3) is located behind the second lens unit, and a distance between said exit pupil (G) and an apex (F) located on a rear optical surface of a last lens in said second lens unit is not more than 30mm.

4. The optical adapter of claim 3, **characterized in that** the first lens unit and the second lens unit are convex lens unit, and a focal length of said first lens unit is greater than a focal length of said second lens unit.

5. The optical adapter of claim 3, **characterized in that** a ratio of focal length between the first lens unit and the second lens unit is greater than 3.

6. The optical adapter of claim 2, **characterized in that** a rotating part (52) is disposed on the body housing (5) for rotating said the plurality of right angle prisms, said plurality of right angle prisms being connected to said rotating part (52).

7. The optical adapter of claim 6, **characterized in that** a scale for indicating a rotational angle of the rotating part (52) is provided on the body housing (5) and/or on the rotating part (52).

8. The optical adapter of claim 2, **characterized in that** the optical image rotating unit being connected to an outside of the body housing (5).

9. The optical adapter of claim 8, **characterized in that** when the optical adapter (B) is connected to the beam splitter (A), the optical image rotating unit is adapted to be disposed between the beam splitter (A) and the one end of the body housing (5).

10. The optical adapter of claim 8, **characterized in that** when the optical adapter (B) is connected to the digital camera equipment (C), the optical image rotating unit is adapted to be disposed between the digital camera equipment (C) and at said another end of the body housing (5).

11. The optical adapter of claim 1, **characterized in that** the optical adapter (B) further comprises a group of mirrors located on the optical path, said group of mirrors including at least one reflecting element which comprises a mirror, a pentaprism, a right angle prism and a right angle roof prism.

12. The optical adapter of claim 11, **characterized in that** the group of mirrors comprising at least three Right Angle Prisms when the plurality of right angle prisms comprises one Right Angle Prism.

13. A microscope optical adapter (B) for connecting between a beam splitter (A) of a microscope (D) and a digital camera equipment (C), the microscope optical adapter (B) comprising:
an optical image rotating unit for adjusting a direction of an optical image on a photosensitive unit of the digital camera equipment (C),
**characterized in that** said optical image rotating unit comprises a plurality of right angle prisms (41) being four right angle prisms (41-2, 41-3, 41-4) which is configured to be fixed on an optical path;
wherein, an orientation of the optical image is adjusted by rotating the plurality of right angle prisms while maintaining a same relative rotation angle between adjacent right angle prisms..

14. The microscope optical adapter of claim 13, **characterized in that** the microscope (D) comprises a surgical microscope or an ophthalmic slit lamp microscope and the digital camera equipment (C) comprises mobile phones, tablet computers, cameras, video cameras with image capturing function.

15. A method for adjusting a direction of an optical image using a microscope optical adapter (B) in a microscope (D), the method comprising the steps of
connecting the microscope optical adapter (B) with a beam splitter (A) of the microscope (D) on its light incident end;
connecting a digital camera equipment (C) to a light emitting end of said microscope optical adapter (B);
forming an optical image on a photosensitive unit of the digital camera equipment (C),
wherein:
the microscope optical adapter (B) comprises an optical image rotating unit for adjusting a direction of an optical image on a photosensitive unit of the digital camera equipment (C), **characterized in that**
the optical image rotating unit comprises a plurality of right angle prisms (41) being four right angle prisms (41-2, 41-3, 41-4) configured to be rotatable around optical axis independently with respect to the beam splitter,
when a light emitting direction of the microscopic optical adapter (B) is changed by rotation of the optical image rotating unit, the optical image formed by the light emitted from different emission directions on the photosensitive unit of said digital camera equipment (C) can be aligned with the image observed by said microscope without an adjustment of computer software;
wherein, an orientation of the optical image is adjusted by rotating the plurality of right angle prisms while maintaining a same relative rotation angle between adjacent right angle prisms.

16. The method of claim 15, **characterized in that** the image forming step comprises a step of passing light from the beam splitter (A) of the microscope (D) respectively through the plurality of right angle prisms and a group of mirrors located on an optical path to form the optical image on the photosensitive unit of the digital camera equipment (C).

17. The method of claim 15, **characterized in that** the microscope (D) comprises a surgical microscope or an ophthalmic slit lamp microscope and the digital camera equipment (C) comprises mobile phones, tablet computers, cameras, video cameras with image capturing function.

## Patentansprüche

1. Optischer Adapter (B) zum Verbinden zwischen einem Strahlteiler (A) eines Operationsmikroskops oder eines ophthalmologischen Spaltlampenmikroskops und einer Digitalkameraausrüstung (C), der optische Adapter (B) umfassend:
eine Linsengruppe (3), die sich in dem Strahlengang befindet; und
eine Dreheinheit für optisches Bild, die konfiguriert ist, um eine Richtung eines optischen Bildes auf einer lichtempfindlichen Einheit der Digitalkameraausrüstung (C) anzupassen,
**dadurch gekennzeichnet, dass** die Dreheinheit für optisches Bild eine Vielzahl von rechtwinkligen Prismen (41) umfasst, die vier rechtwinklige Prismen (41-2, 41-3, 41-4) sind, die sich in dem Strahlengang befinden, wobei die Vielzahl von rechtwinkligen Prismen konfiguriert sind, um in Bezug auf den Strahlteiler unabhängig um eine optische Achse drehbar zu sein;
wobei eine Ausrichtung des optischen Bilds durch Drehen der Vielzahl von rechtwinkligen Prismen angepasst wird, während ein gleichbleibender relativer Drehwinkel zwischen angrenzenden rechtwinkligen Prismen beibehalten wird.

2. Optischer Adapter nach Anspruch 1, **dadurch gekennzeichnet, dass** der optische Adapter (B) ferner ein Körpergehäuse (5) umfasst, das die Linsengruppe (3) umschließt, wobei ein Ende des Körpergehäuses (5) mit dem Strahlteiler (A) verbunden ist, während ein anderes Ende des Körpergehäuses (5) mit der Digitalkameraausrüstung (C) verbunden ist.

3. Optischer Adapter nach Anspruch 1, **dadurch gekennzeichnet, dass** die Linsengruppe (3) mindestens eine erste Linseneinheit zum Bündeln eines von dem Strahlteiler (A) ausgehenden Lichts zu einem realen Bild (E) und eine zweite Linseneinheit zum Projizieren des realen Bildes (E) auf die lichtempfindliche Einheit der Digitalkameraausrüstung (C) umfasst, die zweite Linseneinheit an einem gegenüberliegenden Ende des Strahlengangs im Vergleich zu der ersten Linseneinheit angeordnet ist, wobei sich eine Austrittspupille (G) der Linsengruppe (3) hinter der zweiten Linseneinheit befindet, und ein Abstand zwischen der Austrittspupille (G) und einem Scheitelpunkt (F), der sich auf einer hinteren optischen Oberfläche einer letzten Linse in der zweiten Linseneinheit befindet, nicht mehr als 30 mm ist.

4. Optischer Adapter nach Anspruch 3, **dadurch gekennzeichnet, dass** die erste Linseneinheit und die zweite Linseneinheit konvexe Linseneinheiten sind, und eine Brennweite der ersten Linseneinheit größer ist als die Brennweite der zweiten Linseneinheit.

5. Optischer Adapter nach Anspruch 3, **dadurch gekennzeichnet, dass** ein Verhältnis einer Brennweiten zwischen der ersten Linseneinheit und der zweiten Linseneinheit größer ist als 3.

6. Optischer Adapter nach Anspruch 2, **dadurch gekennzeichnet, dass** an dem Körpergehäuse (5) ein Drehteil (52) zum Drehen der Vielzahl von rechtwinkligen Prismen angeordnet ist, wobei die Vielzahl von rechtwinkligen Prismen mit dem Drehteil (52) verbunden ist.

7. Optischer Adapter nach Anspruch 6, **dadurch gekennzeichnet, dass** an dem Körpergehäuse (5) und/oder an dem Drehteil (52) eine Skala zum Angeben eines Drehwinkels des Drehteils (52) bereitgestellt ist.

8. Optischer Adapter nach Anspruch 2, **dadurch gekennzeichnet, dass** die Dreheinheit für optisches Bild mit einer Außenseite des Körpergehäuses (5) verbunden ist.

9. Optischer Adapter nach Anspruch 8, **dadurch gekennzeichnet, dass**, wenn der optische Adapter (B) mit dem Strahlteiler (A) verbunden ist, die Dreheinheit für optisches Bild angepasst ist, um zwischen dem Strahlteiler (A) und einem Ende des Körpergehäuses (5) angeordnet zu sein.

10. Optischer Adapter nach Anspruch 8, **dadurch gekennzeichnet, dass**, wenn der optische Adapter (B) mit der Digitalkameraausrüstung (C) verbunden ist, die Dreheinheit für optisches Bild angepasst ist, um zwischen der Digitalkameraausrüstung (C) und an dem anderen Ende des Körpergehäuses (5) angeordnet zu sein.

11. Optischer Adapter nach Anspruch 1, **dadurch gekennzeichnet, dass** der optische Adapter (B) ferner eine Gruppe von Spiegeln umfasst, die sich in dem Strahlengang befinden, wobei die Gruppe von Spiegeln mindestens ein reflektierendes Element beinhaltet, das einen Spiegel, ein Pentaprisma, ein rechtwinkliges Prisma und ein rechtwinkliges Dachprisma umfasst.

12. Optischer Adapter nach Anspruch 11, **dadurch gekennzeichnet, dass** die Gruppe von Spiegeln mindestens drei Rechtwinkelprismen umfasst, wenn die Vielzahl von rechtwinkligen Prismen ein Rechtwinkelprisma umfasst.

13. Optischer Adapter für Mikroskop (B) zum Verbinden zwischen einem Strahlteiler (A) eines Mikroskops (D) und einer Digitalkameraausrüstung (C), der optische Adapter für Mikroskop (B) umfassend:
eine Dreheinheit für optisches Bild zum Anpassen der Richtung eines optischen Bilds auf einer lichtempfindlichen Einheit der Digitalkameraausrüstung (C),
**dadurch gekennzeichnet, dass** die Dreheinheit für optisches Bild eine Vielzahl von rechtwinkligen Prismen (41) umfasst, die vier rechtwinklige Prismen (41-2, 41-3, 41-4) sind, die konfiguriert sind, um in einem Strahlengang befestigt zu sein;
wobei eine Ausrichtung des optischen Bilds durch Drehen der Vielzahl von rechtwinkligen Prismen angepasst wird, während ein gleichbleibender relativer Drehwinkel zwischen angrenzenden rechtwinkligen Prismen beibehalten wird.

14. Optischer Adapter für Mikroskop nach Anspruch 13, **dadurch gekennzeichnet, dass** das Mikroskop (D) ein Operationsmikroskop oder ein ophthalmologisches Spaltlampenmikroskop umfasst und die Digitalkameraausrüstung (C) Mobiltelefone, Tablet-Computer, Kameras und Videokameras mit Bildaufnahmefunktion umfasst.

15. Verfahren zum Anpassen einer Richtung eines optischen Bilds unter Verwendung eines optischen Adapters für Mikroskop (B) in einem Mikroskop (D), das Verfahren umfassend die folgenden Schritte:
Verbinden des optischen Adapters für Mikroskop (B) mit einem Strahlteiler (A) des Mikroskops (D) an dessen Lichteinfallsende;
Verbinden einer Digitalkameraausrüstung (C) mit einem lichtemittierenden Ende des optischen Adapters für Mikroskop (B);
Bilden eines optischen Bilds auf einer lichtempfindlichen Einheit der Digitalkameraausrüstung (C),
wobei:
der optische Adapter für Mikroskop (B) eine Dreheinheit für optisches Bild zum Anpassen einer Richtung des optischen Bilds auf einer lichtempfindlichen Einheit der Digitalkameraausrüstung (C) umfasst, **dadurch gekennzeichnet, dass** die Dreheinheit für optisches Bild eine Vielzahl von rechtwinkligen Prismen (41) umfasst, die vier rechtwinklige Prismen (41-2, 41-3, 41-4) sind, die konfiguriert sind, um in Bezug auf den Strahlteiler unabhängig um eine optische Achse drehbar zu sein,
wenn eine lichtemittierende Richtung des optischen Adapters für Mikroskop (B) durch Drehen der Dreheinheit für optisches Bild verändert wird, das optische Bild, das durch das Licht gebildet wird, das aus verschiedenen Emissionsrichtungen auf die lichtempfindliche Einheit der Digitalkameraausrüstung (C) emittiert wird, ohne eine Anpassung von Computersoftware mit dem Bild ausgerichtet werden kann, das von dem Mikroskop beobachtet wird;
wobei eine Ausrichtung des optischen Bilds durch Drehen der Vielzahl von rechtwinkligen Prismen angepasst wird, während ein gleichbleibender relativer Drehwinkel zwischen angrenzenden rechtwinkligen Prismen beibehalten wird.

16. Verfahren nach Anspruch 15, **dadurch gekennzeichnet, dass** der Bilderzeugungsschritt einen Schritt eines Durchleitens von Licht von dem Strahlteiler (A) des Mikroskops (D) jeweils durch die Vielzahl von rechtwinkligen Prismen und eine Gruppe von Spiegeln umfasst, die sich in einem Strahlengang befinden, um das optische Bild auf der lichtempfindlichen Einheit der Digitalkameraausrüstung (C) zu bilden.

17. Verfahren nach Anspruch 15, **dadurch gekennzeichnet, dass** das Mikroskop (D) ein Operationsmikroskop oder ein ophthalmologisches Spaltlampenmikroskop umfasst und die Digitalkameraausrüstung (C) Mobiltelefone, Tablet-Computer, Kameras und Videokameras mit Bildaufnahmefunktion umfasst.

## Revendications

1. Adaptateur optique (B) destiné à être connecté entre un séparateur de faisceau (A) d'un microscope chirurgical ou d'un microscope ophtalmique à lampe à fente et un appareil photo numérique (C), l'adaptateur optique (B) comprenant :
un groupe de lentilles (3) situé sur un trajet optique ; et
une unité de rotation d'image optique configurée pour régler une orientation d'une image optique sur une unité photosensible de l'appareil photo numérique (C),
**caractérisé en ce que** ladite unité de rotation d'image optique comprend une pluralité de prismes à angle droit (41), à savoir quatre prismes à angle droit (41-2, 41-3, 41-4) situés sur le trajet optique, dans lequel ladite pluralité de prismes à angle droit étant configurée pour tourner indépendamment autour de l'axe optique par rapport au séparateur de faisceau ;
dans lequel, une orientation de l'image optique est ajustée en faisant tourner la pluralité de prismes à angle droit tout en conservant un même angle de rotation relatif entre les prismes à angle droit adjacents.

2. Adaptateur optique selon la revendication 1, **caractérisé en ce que** l'adaptateur optique (B) comprend en outre un boîtier (5) renfermant le groupe de lentilles (3), dans lequel une extrémité du boîtier (5) étant reliée au séparateur de faisceau (A) tandis qu'une autre extrémité du boîtier (5) est reliée à l'appareil photo numérique (C).

3. Adaptateur optique selon la revendication 1, **caractérisé en ce que** le groupe de lentilles (3) comprend au moins une première unité de lentilles destinée à faire converger la lumière sortante du séparateur de faisceau (A) en une image réelle (E), et une deuxième unité de lentilles destinée à projeter ladite image réelle (E) sur l'unité photosensible de l'appareil photo numérique (C), ladite deuxième unité de lentilles étant disposée à une extrémité opposée du chemin optique par rapport à ladite première unité de lentilles, dans lequel une pupille de sortie (G) dudit groupe de lentilles (3) est située derrière la deuxième unité de lentilles, et la distance entre ladite pupille de sortie (G) et un sommet (F) situé sur une surface optique arrière d'une dernière lentille de ladite deuxième unité de lentilles n'est pas supérieure à 30 mm.

4. Adaptateur optique selon la revendication 3, **caractérisé en ce que** le premier groupe de lentilles et le deuxième groupe de lentilles sont des groupes de lentilles convexes, et **en ce que** la distance focale dudit premier groupe de lentilles est supérieure à la distance focale dudit deuxième groupe de lentilles.

5. Adaptateur optique selon la revendication 3, **caractérisé en ce que** le rapport des distances focales entre la première unité de lentilles et la deuxième unité de lentilles est supérieur à 3.

6. Adaptateur optique selon la revendication 2, **caractérisé en ce qu'**une pièce rotative (52) est disposée sur le boîtier (5) afin de faire tourner ladite pluralité de prismes à angle droit, ladite pluralité de prismes à angle droit étant reliée à ladite pièce rotative (52).

7. Adaptateur optique selon la revendication 6, **caractérisé en ce qu'**une échelle permettant d'indiquer l'angle de rotation de la pièce rotative (52) est prévue sur le boîtier (5) et/ou sur la pièce rotative (52).

8. Adaptateur optique selon la revendication 2, **caractérisé en ce que** l'unité de rotation d'image optique est reliée à l'extérieur du boîtier (5).

9. Adaptateur optique selon la revendication 8, **caractérisé en ce que**, lorsque l'adaptateur optique (B) est connecté au séparateur de faisceau (A), l'unité de rotation d'image optique est adaptée pour être disposée entre le séparateur de faisceau (A) et l'une des extrémités du boîtier (5).

10. Adaptateur optique selon la revendication 8, **caractérisé en ce que**, lorsque l'adaptateur optique (B) est connecté à l'appareil photo numérique (C), l'unité de rotation d'image optique est adaptée pour être disposée entre l'appareil photo numérique (C) et ladite autre extrémité du boîtier (5).

11. Adaptateur optique selon la revendication 1, **caractérisé en ce que** l'adaptateur optique (B) comprend en outre un ensemble de miroirs situés sur le trajet optique, ledit ensemble de miroirs comprenant au moins un élément réfléchissant qui comprend un miroir, un pentaprisme, un prisme à angle droit et un prisme en toit à angle droit.

12. Adaptateur optique selon la revendication 11, **caractérisé en ce que** le groupe de miroirs comprend au moins trois prismes à angle droit lorsque la pluralité de prismes à angle droit comprend un prisme à angle droit.

13. Adaptateur optique pour microscope (B) destiné à être connecté entre un séparateur de faisceau (A) d'un microscope (D) et un appareil photo numérique (C), l'adaptateur optique pour microscope (B) comprenant :
une unité de rotation d'image optique destinée à régler une orientation d'une image optique sur une unité photosensible de l'appareil photo numérique (C),
**caractérisé en ce que** ladite unité de rotation d'image optique comprend une pluralité de prismes à angle droit (41), à savoir quatre prismes à angle droit (41-2, 41-3, 41-4) configuré pour être fixé sur un trajet optique;
dans lequel une orientation de l'image optique est ajustée en faisant tourner la pluralité de prismes à angle droit tout en conservant un même angle de rotation relatif entre les prismes à angle droit adjacents.

14. Adaptateur optique pour microscope selon la revendication 13, **caractérisé en ce que** le microscope (D) comprend un microscope chirurgical ou un microscope ophtalmologique à lampe à fente et que l'appareil photo numérique (C) est intégré dans des téléphones portables, des tablettes, des appareils photo et des caméras vidéo dotés d'une fonction de capture d'images.

15. Procédé destiné au réglage d'une orientation d'une image optique à l'aide d'un adaptateur optique (B) dans un microscope (D), le procédé comprenant les étapes suivantes :
la connexion de l'adaptateur optique pour microscope (B) à un séparateur de faisceau (A) du microscope (D) au niveau de son extrémité d'incidence de la lumière ;
la connexion d'un appareil photo numérique (C) à l'extrémité émettrice de lumière dudit adaptateur optique pour microscope (B) ;
la formation d'une image optique sur un dispositif photosensible de l'appareil photo numérique (C),
dans lequel :
l'adaptateur optique pour microscope (B) comprend une unité de rotation d'image optique destinée à régler une orientation d'une image optique sur une unité photosensible de l'appareil photo numérique (C), **caractérisé en ce que** l'unité de rotation d'image optique comprend une pluralité de prismes à angle droit (41), à savoir quatre prismes à angle droit (41-2, 41-3, 41-4) configurés pour pouvoir tourner autour de l'axe optique indépendamment du séparateur de faisceau,
lorsque la direction d'émission de la lumière de l'adaptateur optique microscopique (B) est modifiée par la rotation de l'unité de rotation de l'image optique, l'image optique formée par la lumière émise selon différentes directions d'émission sur l'unité photosensible dudit équipement photographique numérique (C) peut être alignée avec l'image observée par ledit microscope sans qu'il soit nécessaire de procéder à un réglage du logiciel informatique ;
dans lequel une orientation de l'image optique est ajustée en faisant tourner la pluralité de prismes à angle droit tout en conservant un même angle de rotation relatif entre les prismes à angle droit adjacents.

16. Procédé selon la revendication 15, **caractérisé en ce que** l'étape de formation d'image comprend une étape consistant à faire passer la lumière provenant du séparateur de faisceau (A) du microscope (D) respectivement à travers la pluralité de prismes à angle droit et un ensemble de miroirs situés sur un chemin optique afin de former l'image optique sur l'unité photosensible de l'appareil photo numérique (C).

17. Procédé selon la revendication 15, **caractérisé en ce que** le microscope (D) comprend un microscope chirurgical ou un microscope ophtalmologique à lampe à fente et que l'appareil photo numérique (C) est intégré dans des téléphones portables, des tablettes, des appareils photo et des caméras vidéo dotés d'une fonction de capture d'images.
